# EUROPÄISCHE PATENTSCHRIFT

(11) **EP 1 252 186 B1**
(45) Veröffentlichungstag und Bekanntmachung des Hinweises auf die Patenterteilung: **11.01.2006**
(21) Anmeldenummer: 01927660.9
(22) Anmeldetag: 09.02.2001
(51) Int. Cl.: C07K 14/475, G01N 33/58, G01N 33/68, G01N 33/84

(54) **VERWENDUNG VON NEUREGULIN-BETA ALS INDIKATOR UND/ODER TARGET**
USE OF NEUREGULIN-BETA AS AN INDICATOR AND/OR TARGET
UTILISATION DE NEUREGULINE-BETA EN TANT QU'INDICATEUR ET/OU CIBLE

(30) Priorität: 11.02.2000 DE 10006174; 11.02.2000 DE 10006175; 15.11.2000 US 248224 P
(43) Veröffentlichungstag der Anmeldung: 30.10.2002
(73) Patentinhaber: ProteoSys AG, 55129 Mainz (DE)
(72) Erfinder: SCHRATTENHOLZ, André, 55129 Mainz (DE)
(74) Vertreter: Weiss, Wolfgang
(86) Internationale Anmeldenummer: PCT/EP2001/001424
(87) Internationale Veröffentlichungsnummer: WO 2001/058948

(56) Entgegenhaltungen:
- WO-A-92/20798
- WO-A1-99/18976
- EILAM R. ET AL.: "Activity-dependent regulation of Neu Differentiation Factor/Neuregulin expression in rat brain" PROCEEDINGS OF THE NATIONAL ACADEMY OF SCIENCES OF THE USA, Bd. 95, Februar 1998 (1998-02), Seiten 1888-1893, XP002187768
- WEN D. ET AL.: "Structural and functional aspects of the multiplicity of Neu Differentiation Factors" MOLECULAR AND CELLULAR BIOLOGY, Bd. 14, Nr. 3, März 1994 (1994-03), Seiten 1909-1919, XP002064746 ISSN: 0270-7306
- CHEW LI-JIN AND GALLO V.: "Regulation of ion channel expression in neural cells by hormones and growth factors" MOLECULAR NEUROBIOLOGY, Bd. 18, Nr. 3, Dezember 1998 (1998-12), Seiten 175-225, XP001053101
- BURGESS T.L. ET AL: 'Biosynthetic processing of neu Differentiation Factor' THE JOURNAL OF BIOLOGICAL CHEMISTRY Bd. 270, Nr. 32, 11 August 1995, Seiten 19188 - 19196
- LU H.S. ET AL: 'Post-translational processing of membrane-associated neu Differentiation Factor proisoforms expressed in mammalian cells' THE JOURNAL OF BIOLOGICAL CHEMISTRY Bd. 270, Nr. 9, 03 M{rz 1995, Seiten 4775 - 4783
- BEN-BARUCH N.; YARDEN Y.: 'Neu Differentiation Factors: a family of alternatively spliced neuronal and mesenchymal factors' PROCEEDINGS OF THE SOCIETY FOR EXPERIMENTAL BIOLOGY AND MEDICINE Bd. 206, Nr. 3, 1994, Seiten 221 - 227
- OZAKI M. ET AL: 'Neuregulin-beta induces expression of an NMDA-receptor subunit' NATURE Bd. 390, Dezember 1997, Seiten 691 - 694
- HO WEI-HSIEN ET AL: 'Sensory and Motor neuron-Derived Factor. A novel Heregulin variant highly expressed in sensory and motor neurons' THE JOURNAL OF BIOLOGICAL CHEMISTRY Bd. 270, Nr. 24, 16 Juni 1995, Seiten 14523 - 14532
- NiceProt View of Swiss-Prot: Q15491 Compute pI/MW of SMDF_HUMAN (Q15491)
- LU H.S. ET AL: 'Studies on the structure and function of glycosylated and nonglycosylated neu Differentiation Factors. Similarities and differences of the alpha and beta isoforms' THE JOURNAL OF BIOLOGICAL CHEMISTRY Bd. 270, Nr. 9, 03 M{rz 1995, Seiten 4784 - 4791

### SEQUENZPROTOKOLL

<110> PROTEOSYS AG
<120> Verwendung von Neuregulin-β als Indikator und/oder Target
<130> 23709PWO_AT
<140> PCT/EP 01/01424
   <141> 2001-02-09
<150> DE 100 06 175.3
   <151> 2000-02-11
<150> DE 100 06 174.5
   <151> 2000-02-11
<150> US 60/248,224
<151> 2000-11-15
<160> 11
<170> PatentIn Ver. 2.1
<210> 1
   <211> 5
   <212> PRT
   <213> Rattus norvegicus
<400> 1
   Met Ser Glu Arg Lys
    1               5
<210> 2
   <211> 6
   <212> PRT
   <213> Rattus norvegicus
<400> 2
   Gly Lys Gly Lys Lys Lys
    1               5
<210> 3
   <211> 6
   <212> PRT
   <213> Rattus norvegicus
<400> 3
   Gln Lys Leu His Asp Arg
    1               5
<210> 4
   <211> 7
   <212> PRT
   <213> Rattus norvegicus
<400> 4
   Ile Gln Lys Lys Pro Gly Lys
   1 5
<210> 5
   <211> 7
   <212> PRT
   <213> Rattus norvegicus
<400> 5
   Asn Lys Pro Glu Asn Ile Lys
   1 5
<210> 6
   <211> 8
   <212> PRT
   <213> Rattus norvegicus
<400> 6
   Ala Glu Glu Leu Tyr Gln Lys Arg
   1 5
<210> 7
   <211> 10
   <212> PRT
   <213> Rattus norvegicus
<400> 7
   Tyr Gly Val Ser Gly Tyr Pro Thr Leu Lys
   1 5 10
<210> 8
   <211> 16
   <212> PRT
   <213> Rattus norvegicus
<400> 8
   Ala Ser Leu Ala Asp Ser Gly Glu Tyr Met Cys Lys Val Ile Ser Lys
   1 5 10 15
<210> 9
   <211> 22
   <212> PRT
   <213> Rattus norvegicus
<400> 9
   Asp Arg Gly Ser Arg Gly Lys Pro Gly Pro Ala Glu Gly Asp Pro Ser
   1 5 10 15
   Pro Ala Leu Pro Pro Arg
   20
<210> 10
   <211> 18
   <212> PRT
   <213> Rattus norvegicus
<400> 10
   Trp Phe Lys Asn Gly Asn Glu Leu Asn Arg Lys Asn Lys Pro Glu Asn
   1 5 10 15
   Ile Lys
<210> 11
   <211> 23
   <212> PRT
   <213> Rattus norvegicus
<400> 11
   Lys Asp Arg Gly Ser Arg Gly Lys Pro Gly Pro Ala Glu Gly Asp Pro
   1 5 10 15
   Ser Pro Ala Leu Pro Pro Arg
   20

## Beschreibung

Die Erfindung betrifft die Verwendung von Neuregulin-β als Indikator und/oder Target in einem Screening-Verfahren zur Identifizierung von Wirkstoffen. Weiterhin betrifft die Erfindung die Verwendung von Neuregulinen, vorzugsweise einer Neuregulin-Isoform mit einem isoelektrischen Punkt im Bereich von pH ≤ 7, insbesondere pH 4,3 bis 5,0, als Target für den Nachweis bzw. die Beeinflussung neuronaler Prozesse, insbesondere für die Beeinflussung des Langzeitgedächtnisses. Neureguline, insbesondere Neuregulin-β und auch Substanzen, die den Status, d.h. die Expression oder/und posttranslationale Modifikation von Neuregulin-β beeinflussen, können daher als Mittel zur Verlaufskontrolle, Behandlung oder/und Linderung von neuronalen Erkrankungen, z.B. von Morbus Alzheimer eingesetzt werden.

Neureguline (auch ARIA, neurogenic differentiation factors, Hereguline und DDF) gehören zu einer Familie weitverbreiteter und bekannter Wachstums- und Differenzierungsfaktoren. Beispielsweise ist in der Publikation von Ozaka M. et al., Nature 1997, Dec 10-25, 390(6661): 691-4 der induzierende Einfluss des Neuregulin-β auf die Expression der NR2C-Untereinheit des NMDA-Rezeptors beschrieben.
**Siehe auch Eilam R. et al., Proc. Natl. Acad. Sci. USA 1998, 95:1888-1893.**
WO99/18976 beschreibt Verfahren zur Behandlung und/oder Prophylaxe von neurologischen Erkrankungen, umfassend die Verabreichung eines Neuregulins, oder eines Fragments oder Derivats eines Neuregulins, oder einer Nukleinsäure codierend ein Neuregulin oder ein Neuregulinfragment oder -derivat an einen Patienten. In WO99/18976 werden lediglich Untersuchungen auf Genebene durchgeführt. Da aus einem einzigen Gen durch Modifikationen auf Transkript- oder/und Proteinebene bis zu mehr als 100 unterschiedliche molekulare Proteinspezies entstehen können, sind die Angaben in WO99/18976 nicht ausreichend, um diejenigen Proteinspezies identifizieren zu können, die tatsächlich für neurologische Prozesse relevant sind.

W098/5561 1 beschreibt die Verwendung eines 15 bp-langen Neuregulin-Response-Elements in therapeutischen Verfahren und Screening-Verfahren zur Identifizierung von Wirkstoffen.

Seit einiger Zeit sind die Techniken der Proteom-Analyse eingeführt. Unter dem Begriff "Proteom" ist die Gesamtheit aller exprimierten Proteine einer Zelle, eines Gewebes oder eines Organismus zu verstehen (siehe: "From Genome to Proteome" von Michael J. Dunn, 2000, Wiley/VCH, Weinheim; "Proteome Research, Two-Dimensional Gel-Elektrophoresis and Identification Methods" von Thierry Rabilloud, 2000, Springer-Verlag, Berlin; siehe auch: "Proteome Research: New Frontiers in Functional Genomics" von Marc R. Wilkins, u.a., 1997, Springer-Verlag, Berlin). Die Techniken der Proteom-Analyse sind die 2-D-PAGE (zweidimensionale Gelelektrophorese) zur Auftrennung der einzelnen Proteine aus einer komplexen biologischen Probe und Methoden der Massenspektrometrie, insbesondere MALDI-TOF-MS (matrix-assisted laser desorption ionisation time of flight mass spectrometry) und ESI-MS (electro spray ionisation mass spectrometry) (siehe: "Mass Spectrometry: Principles and Applications", Edmond De Hoffmann, Jean Charette, Vincent Stroobant, Jul Trottier (August 1996), John Wiley & Sons, ISBN: 0471966975).

Verfahren und Vorrichtungen zur Identifizierung biologisch aktiver Substanzen und ihre Wirkung auf lebende Zellen sind in US-Patent 5,721,135 beschrieben. US-Patent 5,089,385 beschreibt ein Verfahren zur Kultivierung von Zellen in einem Durchfluss-Zellkultivierungssystem. US-Patent 5,134,062 beschreibt ein Verfahren zur Diagnose neuronaler Abnormitäten und zur Identifizierung potenzieller therapeutischer Wirkstoffe zu deren Behandlung. DE-OS 19735926 beschreibt eine Anordnung zur Messung der NADH-Eigenfluoreszenz von Zellkulturen. WO90/05179 offenbart eine perfundierbare Zellkultur-Vorrichtung zur Expandierung und Kultivierung biologischer Zellen.

Während auf dem Gebiet der Genome die entsprechenden AnalyseTechniken wohl als ausgereift gelten können, fehlen auf dem Gebiet der

Proteome nach wie vor Techniken, die es einem erlauben, neben der eigentlichen Analyse von Proteinen und deren Bruchstücken auch die im Zusammenhang mit physiologischen Vorgängen stattfindenden komplexen zeitlichen Abläufe der verschiedensten Akionen und Wechselwirkungen auch zeitlich aufgelöst zu erfassen und somit Rückschlüsse auf die eigentlichen physiologischen Vorgänge und die zugrundeliegenden Interaktionen und deren Partner zu ziehen. Auf diesem Wege wäre es möglich, gezielt pharmazeutische Wirkstoffe zu testen.

In diesem Zusammenhang wird erfindungsgemäß unter dem Begriff "Effektraum-/Effektorraum-Analyse" Folgendes verstanden:

Ein Effektraum ist definiert durch ein Koordinatensystem, bei dem jede Koordinate die möglichen Größen oder Amplituden eines Effekts repräsentieren, z.B. die möglichen Konzentrationen des intrazellulären Calciums, die unter physiologischen und pathophysiologischen Umständen in einer Nervenzelle erreicht werden können. Alle in Betracht zu ziehenden Effekte bilden dann den Effektraum, bei drei möglichen Effekten ist ein solcher Effektraum dreidimensional, bei n Effekten n-dimensional.
Gleiches gilt für die Betrachtung der Konzentrationen von Effektoren, z.B. Neurotransmittern etc. Die "Effektraum-/Effektorraum-Analyse" integriert experimentelle Bestimmungen/Messungen zu den Konditionen von Effekten und Effektoren in einem gegebenen System im Hinblick auf den medizinisch/physiologisch interessanten Übergang zwischen Effektraum und Effektorraum, der durch Affinitäten, Reaktions- und Transport- und andere kinetische Parameter definiert wird.

Beim Screening von Wirkstoffen ist mindestens ein Parameter, also ein Indikator oder ein Target, für den betrachteten Effektraum eines Zellsystems notwendig, um das Screening als solches zu ermöglichen. Daneben können jedoch unabhängig hiervon bzw. als quasi Vorstufe zum Screening durch genaue Beobachtung und Auswertung der einzelnen Ergebnisse mittels Korrelationsanalyse auch Indikatoren und/oder Targets ermittelt werden. Unter Indikatoren im erfindungsgemäßen Sinne werden Stoffe bezeichnet, die in einem Effektraum einen Parameter darstellen, da sie bei zu untersuchenden physiologischen Vorgängen aktiv in Erscheinung treten, beispielsweise durch Konzentrationsänderung und somit quasi sichtbar und effektraumgebunden bzw. systemgebunden eben einen Indikator darstellen. Im Allgemeinen werden die zu untersuchenden Zellsysteme mit einer bestimmten Substanz stimuliert, beispielsweise mit Glutamat, die Ergebnisse aufgezeichnet, miteinander korreliert und ggfs. neue Indikatoren herausgefunden. Die Indikatoren stehen dabei in der Regel nicht direkt, sondern über mehrere Schritte indirekt kausal mit den Stimulierungen in Verbindung. Unter dem Begriff Target werden erfindungsgemäß Stoffe bezeichnet, die in einem Effektraum einen Parameter darstellen, da sie bei zu untersuchenden physiologischen Vorgängen aktiv in Erscheinung treten, beispielsweise durch Konzentrationsänderung und somit quasi sichtbar und effektraumgebunden bzw. systemgebunden eben einen speziellen Typ von Indikator darstellen. Im Allgemeinen werden die zu untersuchenden Zellsysteme mit einer bestimmten Substanz stimuliert, beispielsweise mit Glutamat, die Ergebnisse aufgezeichnet, miteinander korreliert und ggfs. diese neuen speziellen Indikatoren herausgefunden. Diese speziellen Indikatoren, nämlich die Targets, stehen dabei in der Regel nicht über mehrere Schritte indirekt - wie bei den eigentlichen Indikatoren - sondern direkt kausal mit den Stimulierungen in Verbindung. Somit ist jeder Indikator potentiell ein Target und umgekehrt; dies hängt selbstverständlich vom zu untersuchenden Zellsystem und dem Effektraum ab.

Ein Problem der vorliegenden Erfindung ist es daher, eine solche Zielstruktur in einem Screening-Verfahren für Wirkstoffe bereitzustellen. Ein weiteres Problem der vorliegenden Erfindung ist es, neue Targets zur Beeinflussung neuronaler Prozesse aufzufinden.

Diese Probleme werden durch den Gegenstand der Anmeldung gelöst. Zunächst geht es erfindungsgemäß um die Verwendung von Neuregulin-β als Indikator und/oder Target in einem Screening-Verfahren zur Identifizierung von Wirkstoffen, insbesondere von Wirkstoffen zur Beeinflussung neuronaler Prozesse. Es konnte überraschenderweise gezeigt werden, dass Neuregulin-β als Indikator und/oder Target in einem solchen Screening-Verfahren eingesetzt werden kann. Das Screening-Verfahren kann für Wirkstoffe zur Beeinflussung von von Glutamatrezeptoren vermittelten Calcium-Konzentrationsänderungen (unter Calcium ist hier Ca²⁺ zu verstehen) eingesetzt werden und zur Erfassung von multidimensionalen Funktionsdaten für die Effektraum-/Effektorraum-Analyse von Wirkstoffen dienen.

Weiterhin geht es erfindungsgemäß um die Verwendung von Neuregulin-β als Indikator bzw. Target in Verfahren zum Nachweis bzw. zur Beeinflussung neuronaler Prozesse. Die Verwendung als Target beinhaltet dabei eine Modulation der Aktivität, Menge und/oder des molekularen Status hinsichtlich posttranslationaler Modifikationen (PTM) von Neuregulin-β in einer Zielzelle, z.B. einem Zielgewebe oder Zielorgan. Vorzugsweise sind die Zielzellen neuronale Zellen.

Schließlich betrifft die Erfindung auch neue Neuregulin-β Isoformen, z.B. PTM-Varianten, die isoelektrische Punkte von pH ≤ 7 und besonders bevorzugt isoelektrische Punkte im pH-Bereich zwischen 4,3 und 5,0, insbesondere zwischen pH 4,5 und 4,7, aufweisen. Diese neuen Neuregulin-β Isoformen und PTM-Varianten sind von besonderer Bedeutung für neuronale Prozesse.

Bei dem Verfahren zur Erfassung von multidimensionalen Funktionsdaten für die Effekt-/Effektraum-Analyse von Wirkstoffen werden zunächst in eine Fließzell-Vorrichtung eingebrachte und auf Substraten aufgebrachte Zellkultursysteme mit jeweils geeigneten Markierungssubstanzen, z.B. Fluoreszenzfarbstoffen und/oder mit Isotopengemischen verschiedener Elemente inkubiert. Anschließend werden die Zellkultursysteme stimuliert und zeitlich synchron die in Abhängigkeit von den Stimulationsbedingungen resultierenden Fluoreszenzsignale direkt am Zellkultursystem zeitlich aufgelöst erfasst und/oder die Isotopengemische im Perfusat zeitlich aufgelöst erfasst und/oder die Isotopengemische direkt an den Zellkultursystemen zeitlich integral erfasst, wobei eine synchronisierte Fraktionierung und weitergehende Analyse des Perfusates, eine Charakterisierung und Identifizierung von sich unter den gewählten Bedingungen verändernden und/oder spezifisch verhaltenden Proteinen der Zellkultursysteme und schließlich eine Integration der gewonnenen multidimensionalen Funktionsdaten für die Effektraum-/Effektorraum-Analyse von Wirkstoffen vorgenommen wird.

Dieses oben beschriebene Verfahren für die Effektraum-/Effektorraum-Analyse und zur Durchführung des Verfahrens geeignete Vorrichtungen sind auch in allgemeiner Form, d.h. nicht in Zusammenhang mit Neuregulin, eine selbständige Erfindung und somit Gegenstand der vorliegenden Anmeldung.

Erfindungsgemäß sind unter den Begriffen "LTP" und "LTD" "Langzeit-Potenzierung" und "Langzeit-Depression" zu verstehen. Es konnte überraschenderweise die direkte Beteiligung von Neuregulin-β an neuronalen Langzeit-Prozessen gezeigt werden, die durch glutamatinduzierte Änderungen der intrazellulären Calcium-Konzentrationen über die NMDA-Rezeptoren vermittelt wird (allgemeine Literaturhinweise: Anwyl R., "The role of amino acid receptors in synaptic plasticity", in: Cortical plasticity, LTP and LTD (Fazeli M.S. & Collingride G.L. eds.) Bios. Scientific Publishers, Oxford, 9-28 (1996); Cormier, R.J., Mauk, M.D. & Kelly, P. "Glutamate iontophoresis induces long-term potentiation in the absence of evoked presynaptic activity", Neuron 10, 907-919 (1993); Malgaroli, A. & Tsien, R.W. "Glutamate-induced long-term potentiation of the frequency of miniature synaptic currents in cultured hippocampal neurons", Nature 357, 134-139 (1992); Soskic-V, Gorlach M., Poznanovic S., Boehmer F.D., Godovac-Zimmermann J., "Functioncal proteomics analysis of signal transduction pathways of the platelet-derived growth factor beta receptor", Biochemistry 38, 1757-64 (1999); Wong et al., "The anticonvulsant MK-801 is a potent NMDA antagonist", Proc. Natl Acad Sci USA 83, 7104-7106 (1986)).

Erfindungswesentlich ist die Tatsache, dass lediglich und auf überraschende Art und Weise nur mit Hilfe der speziellen und synchronen Zusammenschaltung der oben genannten Arbeitsschritte es erstmals möglich ist, die hohen Anforderungen an die Reproduzierbarkeit der einzusetzenden Analysetechniken für solch zu untersuchende Zellsysteme zu gewährleisten (es handelt sich um chaotische Systeme). Eine sukzessive Anwendung der einzelnen Analyseschritte würde nicht zu den gewünschten auswertbaren Ergebnissen führen.

Zunächst ist es vorteilhaft, wenn das Neuregulin-β als Indikator und/oder Target zur Beeinflussung von LTP, LTD, epileptoformen und/oder epileptogenen Ereignissen und/oder erregungs-toxischen Phänomenen eingesetzt wird, da aus den unten gezeigten Ergebnissen hervorgeht, dass Neuregulin-β ein charakteristischer Schlüsselfaktor bei den gedächtnisrelevanten physiologischen Phänomenen LTP/LTD ist.

Es ist vorteilhaft, wenn die Zellkultursysteme auf in die Fließzell-Vorrichtung zu applizierende Objektträger kultiviert werden, da nur so gewährleistet ist, dass die Zellen auf schnellstmöglichem Weg intakt und ohne Ablösungen den Funktionstests zugeführt wird.

Weiterhin ist es von Vorteil, wenn die in die Fließzell-Vorrichtung eingebrachte Zellkultursysteme zunächst mikroskopisch charakterisiert werden, um die basalen Voraussetzungen für weitere funktionelle Anwendungen zu überprüfen.

Die mikroskopische Charakterisierung ist vorteilhafterweise eine morphologische Charakterisierung im Phasenkontrast oder eine videomikroskopische Erfassung vom Zellverhalten unter Einfluss verschiedener physikalischer Parameter und/oder in Abhängigkeit von den Substraten, da die genannten Parameter am besten eine schnelle Einschätzung der Intaktheit und Lebensfähigkeit der Zellen erlauben.

Bei Verwendung von Fluoreszenzmarkierungen wird die Inkubation in vorteilhafter Weise, da in der Praxis bewährt, mittels Fura-2, Indo-1, Bis-Fura-2, Quin-2 und Derivate, Mag-Fura-2, Mag-Fura-5 und/oder Mag-Indo-1 als Fluoreszenzfarbstoff durchgeführt. Darüber hinaus sind noch weitere Fluoreszenzfarbstoffe wie beispielsweise Anthracen-9,10-dipropionsäure einsetzbar. Bei den zuerst genannten Namen handelt es sich um Trivialnamen allgemein bekannter Fluoreszenzfarbstoffe, die auch im Handbook of Fluorescent Probes and Research Chemicals, 6^{th} edition, R.P. Haugland, 1996, Molecular Probes, Eugene, Oregon, USA aufgeführt sind.

Bei Verwendung von Isotopenmarkierungen wird die Inkubation vorteilhafterweise mittels eines Isotopengemisches in Form von Ionen oder Verbindungen mindestens eines der Elemente Wasserstoff, Calcium, Magnesium, Zink, Mangan, Selen, Kupfer, Cadmium, Cobalt, Kohlenstoff, Stickstoff, Eisen, Sauerstoff, Schwefel und Phosphor durchgeführt, da diese Elemente die wichtigsten Elemente sind, die in lebenden Systemen vorkommen.

Es ist von Vorteil, wenn die Stimulation mittels Liganden von glutamatergen, GABA-ergen, cholinergen Liganden durchgeführt wird, da sich diese in der Praxis bewährt haben. Weiterhin sind Liganden für Serotonin-, Catecholamin-, Opioid-, Melatonin-, Peptid-Rezeptoren, spannungsgesteuerte lonen-Kanäle, Transportsysteme für Neurotransmitter, lonen und Metabolite denkbar. Als Agonisten sind insbesondere Acetylcholin und Nicotin zu nennen.

Weiterhin ist es von Vorteil, wenn die zeitlich aufgelöste Erfassung der Isotopengemische im Perfusat mittels ICP-MS und/oder die zeitlich integrale Erfassung der Isotopengemische mittels ICP-MS durchgeführt wird, da es zum einen möglich ist, zeitgleiche extrem empfindliche Konzentrationsmessungen beliebiger Elemente durchzuführen und zum anderen bei Verwendung geeigneter stabiler Isotopenmarker kompartimentspezifische Verteilungen bestimmter Elemente/Ionen zu analysieren.

Außerdem wird in vorteilhafter Weise die weitergehende Analyse des Perfusates mittels Fluoreszenz-HPLC und/oder mit Hilfe von enzymgekoppelten Säulen und/oder elektrochemisch oder fluorometrisch (siehe hierzu: Klapproth H., Reinheimer T., Metzen J., Munch M., Bittinger F., Kirkpatrick C.J., Hohle K.D., Schemann M., Racke K., Wessler I., 1997, Non-neuronal acetylcholine, a signalling molecule synthezised by surface cells of rat and man, Naunyn Schmiedebergs Arch Pharmacol., 355(4): 515-23; oder Rieny J., Tucek S., Vins I., 1992, Sensitive method for HPLC determination of acetylcholine, choline and their analogues using fluorometric detection, J. Neurosci Methods, 41(1): 11 - 17) und/oder massenspektrometrisch, beispielsweise mittels EI-MS (Elektronenstoßlonisations-Massenspektrometrie; Proben werden durch einen Elektronenstrahl ionisiert), FAB-MS (fast atom bombardement mass spectrometry: Proben werden durch Beschuß mit Edelgas-Atomen ionisiert), FD-MS (Feld-Desorptions-Massenspektrometrie; unter dem Einfluss hoher elektrischer Felder werden von einem Draht, auf den eine feste Probe aufgetragen ist, positive Ionen desorbiert und analysiert), MALDI-TOF-MS und ESI-MS durchgeführt, da sich diese Methoden in der Praxis bewährt haben.

Es ist von Vorteil, wenn bei der Analyse das Perfusat mit Fluoreszenzmarkierungen derivatisiert wird, und die Markierungen anschließend über HPLC mit einem Fluoreszenzdetektor identifiziert und quantifiziert werden, da auf diese Weise die erforderlichen hohen Empfindlichkeiten erreicht werden, um in den relativ kleinen Volumina der Fließzell-Vorrichtung die in Frage kommenden Stoffe in einem Arbeitsgang nachweisen (1- 50 Femto-Mole).

Folgende Ausführungsformen haben sich in vorteilhafter Weise in der Praxis bewährt:

Bei der Analyse von Aminosäuren werden diese mit o-Phthaldialdehyd und 2-Mercaptoethanol unter alkalischen Bedingungen derivatisiert und anschließend identifiziert. Alternativ können Aminosäuren auch durch Derivatisierung mit anderen Reagenzien, wie etwa 6-Aminochinolyl-N-hydroxysuccinimidylcarbamat oder anderen Carbamaten oder Phenylisothiocyanaten derivatisiert werden (vlg. z.B. Bidlingmeyer et al., J. Chromatogr. 336 (1984), 93 - 104; lwaki et al., J. Chromatrogr. 407 (1987), 273 - 279; Cohen und Strydom, Anal. Biochem. 174 (1988), 1 - 16; Cohen et al.: "Compositional Protein Analysis Using 6-Aminoquinolyl-N-Hydroxysuccinimidyl Carbamate, a Novel Derivatization Reagent", in: Techniques in Protein Chemistry IV, R.H. Angeletti, Hrsg. (1993), Academic Press, San Diego, CA.; Strydom und Cohen: "Sensitive Analysis of Cystine/Cysteine using 6-Aminoquinolyl-N-Hydroxysuccinimidyl Carbamate (AQC) Derivatives", in: Techniques in Protein Chemistry IV, R.H. Angeletti, Hrsg. (1993), Academic Press, San Diego, CA).

Bei der Analyse von Catecholaminen werden diese vorzugsweise mit Benzylamin unter Einfluss von Kaliumhexacyanoferrat zu fluoreszierenden Verbindungen derivatisiert und anschließend identifiziert. Bei der Analyse von Acetylcholin und Cholin werden diese vorzugsweise elektrochemisch mit Hilfe einer enzymgekoppelten Säule detektiert. Bei der Analyse von Nukleotiden, insbesondere Adenin-Nukleotiden, werden diese vorzugsweise durch Reaktion mit Chloracetaldehyd derivatisiert und als Etheno-Verbindungen detektiert.

Die Charakterisierung und Identifizierung von sich unter den gewählten Bedingungen verändernden und/oder spezifisch verhaltenden Proteinen der Zellkultursysteme wird mittels 2-D-PAGE und MALDI-TOF-MS und/oder ESI-MS durchgeführt, da sich diese Methoden in der Praxis bewährt haben.

Es ist von Vorteil, wenn alle Messungen temperaturkontrolliert durchgeführt werden, da Änderungen der Temperatur um 0,5 - 1 °C, insbesondere im Zentralnervensystem, eine wichtige physiologische Rolle spielen (siehe: Kavanau, J.L., "Memory, sleep and the evolution of mechanisms of synaptic efficacy maintenance", 1997, Neuroscience, 79, 7 - 44).

Bei dem erfindungsgemäßen Verfahren kann das Zellwachstum und Zellverhalten auf und bezüglich für die Zellkultursysteme verwendeten Trägern analysiert werden, so dass in vorteilhafter Weise alle interessierenden Eigenschaften der Trägermaterialien, insbesondere in Bezug auf künstliche Ersatzstoffe in der Implantationsmedizin aber auch in Bezug auf die Authentizität von Zellkultursystemen für ihren jeweiligen Krankheits- oder Gewebe-Modellcharakter, analysiert werden können und folglich eine Validierung der verwendeten Modellsysteme möglich ist.

Aus dem Stand der Technik bekannt sind Fließzell-Vorrichtungen (Firma GlycoTech Corporation; siehe www.Glycotech.com), die insbesondere eine Kammerhöhe von ca. 150 µm aufweisen, die aber für die erfindungsgemäßen Zwecke nicht geeignet sind.

Eine erfindungsgemäße Fließzell-Vorrichtung, insbesondere für den Einsatz in Verfahren zur Erfassung von multidimensionalen Funktionsdaten für die Effektraum-/Effektorraum-Analyse von Wirkstoffen, mit Grundplatte mit Strömungskanal, Oberplatte und Verschlußring, ist dadurch gekennzeichnet, dass die von der Grundplatte mit Strömungskanal und der aufliegenden Deckplatte gebildeten Kammer eine Kammerhöhe von 30 bis 100 µm aufweist. Die individuell einstellbare Kammerhöhe ist in vorteilhafter Weise mittels in mindestens einer der beiden Platten (Ober- und Grundplatte) einzulassenden Stiften, insbesondere aus Metall oder Teflon, zu realisieren.

Mit einer solchen Fließzell-Vorrichtung ist es erstmals möglich durch die spezifische Ausgestaltung, insbesondere niedermolekulare Transmitterfreisetzungen durch neuronale Zellen nachzuweisen. Durch ein besonders günstiges Zellen-/Volumen-Verhältnis ist eine physiologische Scherrate der Flüssigkeit auf den Zellen überraschenderweise überhaupt erst realisierbar. Physiologische Scherraten sind diejenigen Scherraten, wie sie beispielsweise in Blutgefäßen oder anderen biologischen Hohlräumen, in denen sich bewegte Flüssigkeiten befinden, auftreten können (0,1 - 5 Pa). Durch die sehr hoch ausgeprägte Strömungslaminarität sind gleichmäßige Verteilungen von Wirkstoffen und gleichmäßige Bedingungen an nahezu allen Punkten der Durchfließ-Vorrichtung vorhanden. Darüber hinaus ist eine unmittelbare Lyse von Präparaten möglich.

Mit dieser Fließzell-Vorrichtung ist es erstmals möglich, die sehr hohen Anforderungen bezüglich der Empfindlichkeit der abgegebenen Stoffe so zu erfüllen, dass zuverlässige Konzentrationsaussagen bezüglich dieser Stoffe möglich sind (bei zu großen Volumina wären die Konzentrationen der einzelnen Stoffe zu gering).

Zunächst ist es vorteilhaft, wenn die Kammerhöhe der Fließzell-Vorrichtung 30 bis 50 µm beträgt, da so ein für die Mikroskopie optimaler Objektabstand im Hinblick auf hohe Vergrößerungen und große Lichtausbeuten gewährleistet wird. Außerdem wird durch das kleine Volumen der Fließzell-Vorrichtung eine zu große Verdünnung der durch die Zellen abgegebenen Stoffe verhindert; desweiteren ergibt sich durch diese Geometrie eine sehr günstige Reynold-Zahl für laminare Strömung.

In vorteilhafter Weise sind die Zu- und Ableitungen nahezu in der Strömungskanal-Ebene angeordnet, um Turbulenzen zu vermeiden.

In vorteilhafter Weise sind die Innenoberflächen der Kammer nahezu planar ausgebildet, da auf diese Art und Weise gleichmäßige Bedingungen in Bezug auf Druck, Scherraten und die Mikroskopie (Justierung der Ebenen) zu gewährleisten sind.

Weiterhin weist die Fließzell-Vorrichtung zusätzlich ein Objektträger-Aufnahmerahmen zwischen Grund- und Oberplatte auf, um Norm-Objektträger aufnehmen zu können. Dies stellt eine Kompatibilität mit Laborautomaten für die Histologie und/oder lmmuncytochemie sicher.

Der Objektträger-Aufnahmerahmen ist metallisch, um in vorteilhafter Weise hohe mechanische Festigkeit und Verbindungssteifigkeit zu erreichen und eine Sterilisation bei T ≥ + 100 °C zu ermöglichen.

Die Fließzell-Vorrichtung weist vorteilhafterweise eine elastische Dichtung zwischen Grund-und Oberplatte auf, da so ein definierter Abstand und die Dichtigkeit der Kammer gewährleistet sind.

Es ist von Vorteil, wenn die elastische Dichtung aus Silikon besteht, weil dieses Material unter Standardbedingungen autoklavierbar ist und im Hinblick auf die Zellverträglichkeit neutrale Eigenschaften aufweist.

Die Grund- und/oder Oberplatte besteht vorteilhafterweise aus Polycarbonat, da dieses Material Transparenz (UV/Vis) für alle in Frage kommenden mikroskopischen Techniken gewährleistet.

Die Fließzell-Vorrichtung weist in bewährter Weise mehrere nahezu parallel angeordnete Kammern auf, um mehrere Wirkstoffe unter exakt gleichen Bedingungen im Hinblick auf die Zell-Populationen zu testen.

Das erfindungsgemäße Verfahren eignet sich in besonderer Weise zum Screening für Wirkstoffe, insbesondere zur Ermittlung von Targets. Unter dem Begriff "Targets" werden Zielstrukturen verstanden. Hierbei handelt es sich um Proteine oder Metabolite, denen eine entscheidende Schlüsselfunktion bei einem krankheitsrelevanten physiologischen Prozeß zukommt und die sich deswegen besonders als Angriffsziel von Wirkstoffen qualifizieren. Die Identifizierung eines Targets erlaubt die Durchführung weiterer Screening-Verfahren, um Wirkstoffe zu identifizieren, bei denen es sich um Modulatoren, d.h. Aktivatoren oder Inhibitoren des Targets handelt.

Darüber hinaus eignen sich die verschiedenen Träger für Zellkultursysteme im erfindungsgemäßen Verfahren zur Analyse des Zellwachstums und Zellverhaltens.

Die erfindungsgemäße Vorrichtung zur Erfassung von multidimensionalen Funktionsdaten für die Effektraum-/Effektorraum-Analyse von Wirkstoffen, insbesondere zur Durchführung eines oben beschriebenen Verfahrens enthält:
- eine Fließzell-Vorrichtung,
- eine Einrichtung zur Stimulation der Zellkultursysteme,
- eine Einrichtung zur synchronen und zeitlich aufgelösten direkten Erfassung von in Abhängigkeit von den Stimulationsbedingungen resultierenden Fluoreszenzsignalen an den Zellkultursystemen und/oder eine Einrichtung zur zeitlich aufgelösten Erfassung von Isotopengemischen im Perfusat und/oder eine Einrichtung zur zeitlich integralen Erfassung von Isotopengemischen direkt an den Zellkultursystemen,
- eine Einrichtung zur synchronisierten Fraktionierung und weitergehenden Analyse des Perfusates,
- eine Einrichtung zur Charakterisierung und Identifizierung von sich unter den gewählten Bedingungen verändernden und/oder spezifisch verhaltenden Proteinen der Zellkultursysteme und
- eine Einrichtung zur Integration der gewonnenen multidimensionalen Funktionsdaten für die Effektraum-/Effektorraum-Analyse von Wirkstoffen.

Es ist auf Grund der oben beschriebenen vorteilhaften Eigenschaften von Vorteil, wenn die erfindungsgemäße Vorrichtung eine Fließzell-Vorrichtung der oben beschriebenen Art aufweist.

Die folgenden Ausgestaltungen haben sich in der Praxis bewährt und sind aus den zum erfindungsgemäßen Verfahren genannten Gründen als vorteilhaft anzusehen:

Die Einrichtung zur Stimulation der Zellkultursysteme ist ein computergesteuertes Applikationssystem.

Die Einrichtung zur synchronen und zeitlich aufgelösten direkten Erfassung von in Abhängigkeit von den Stimulationsbedingungen resultierenden Fluoreszenzsignalen an den Zellkultursystemen ist eine Fluoreszenzspektroskopie-Einrichtung.

Die Einrichtung zur zeitlich aufgelösten Erfassung von Isotopengemischen im Perfusat ist eine ICP-MS-Einrichtung (siehe hierzu: ICP Spectrometry and Ist Applications von Steve J. Hill (Herausgeber), 2000, CRC Press, ISBN: 0849397391).

Die Einrichtung zur zeitlich integralen Erfassung von Isotopengemischen direkt an den Zellkultursystemen ist eine ICP-MS-Einrichtung.

Die Einrichtung zur synchronisierten Fraktionierung und weitergehenden Analyse des Perfusates weist einen computergesteuertern Fraktionssammler und eine HPLC-Einrichtung mit Fluoreszenzdetektor und/oder eine Einrichtung mit enzymgekoppelten Säulen und/oder eine Einrichtung mit elektrochemischer oder fluorometrischer Detektion und/oder ein Massenspektrometer auf.

Die Einrichtung zur Charakterisierung und ldentifizierung von sich unter den gewählten Bedingungen verändernden und/oder spezifisch verhaltenden Proteinen der Zellkultursysteme umfaßt eine 2-D-PAGE-Einrichtung und eine MALDI-TOF-MS-Einrichtung und/oder ESI-MS-Einrichtung.

Die Einrichtung zur Integration der gewonnenen multidimensionalen Funktionsdaten für die Effektraum-/Effektorraum-Analyse von Wirkstoffen ist eine datenbankgestützte Computer-Einrichtung.

Die Einrichtung zur Analyse des Zellwachstums und des Zellverhaltens auf und bezüglich für die Zellkultursysteme verwendeten Trägern ist eine computergestützte Bildanalyse-Einrichtung mit einem Videomikroskop.

Die Ergebnisse der erfindungsgemäßen Verwendungen können selbstverständlich auch als Zwischenergebnisse für Diagnoseverfahren verwendet werden. Die Ergebnisse dienen somit quasi als Entscheidungsgrundlage für das weitere Vorgehen bzw. bestimmen den weiteren Verlauf innerhalb eines bestimmten Diagnoseverfahrens.

Noch ein weiterer Gegenstand der Erfindung ist die Verwendung von Neuregulin-β als Indikator in einem Verfahren zum Nachweis neuronaler Prozesse. Dieses Verfahren ist vorzugsweise ein diagnostisches Verfahren, umfassend die Bestimmung des Vorhandenseins, der Konzentration und/oder der Aktivität von Neuregulin-β in einer zu untersuchenden Probe (in vitro Diagnose) oder einem zu untersuchenden Organismus (in vivo Diagnose). Ein derartiges Diagnoseverfahren kann beispielsweise einen Nachweis auf Proteinebene, z.B. unter Verwendung von Antikörpern, oder einen Nachweis auf Transkriptebene, z.B. durch Northern Blots, reverse Transkription und/oder Amplifikationsprozesse umfassen. Die Durchführung derartiger Diagnoseverfahren ist dem Fachmann grundsätzlich bekannt.

Weiterhin betrifft die Erfindung die Verwendung von Neuregulin-β als Target in einem Verfahren zur Beeinflussung neuronaler Prozesse. Dieses Verfahren kann eine Veränderung, z.B. eine Verringerung oder Erhöhung, der Konzentration und/oder Aktivität von Neuregulin-β, vorzugsweise im Bereich des Gehirns, des Rückenmarks und/oder der Nervenzellen umfassen. Dieses Verfahren kann die Verabreichung von Neuregulin-β Inhibitoren, z.B. Antikörper oder niedermolekulare Substanzen und/oder die Verabreichung von Neuregulin-β Antisense Nukleinsäuren umfassen, falls eine Verringerung der Neuregulin-β Konzentration und/oder Aktivität gewünscht wird. Andererseits kann eine Verabreichung von Neuregulin-β oder einer dafür kodierenden Nukleinsäure an einen Zielort im Organismus erfolgen, falls eine Erhöhung der Neuregulin-β Menge und/oder Aktivität gewünscht wird. Die Verabreichung von Neuregulin-β kann in freier Form oder assoziiert an einen geeigneten Träger, z.B. an Vehikel wie Mizellen, Liposomen etc. erfolgen. Verabreichung von Neuregulin-β kodierenden Nukleinsäuren kann in Form von so genannter "nackter" DNA oder in Form von Vehikeln wie zuvor angegeben oder alternativ in Form von viralen Vektoren, z.B. Adenoviren, Retroviren etc. erfolgen.

Alternativ oder zusätzlich kann auch eine Verabreichung von Substanzen erfolgen, die die Expression und/oder den Status von Neuregulin-β beeinflussen. Derartige Substanzen, z.B. Inhibitoren oder Aktivatoren, können auf einfache Weise durch das erfindungsgemäße Effektraum/Effektorraum-Analyseverfahren oder durch andere Screening-Verfahren identifiziert werden. Die Erfindung umfasst daher auch solche Substanzen, zB. niedermolekulare Wirkstoffe und/oder biologische Substanzen, z.B. Makromoleküle wie Proteine, Nukleinsäuren etc., und daraus durch Modifikationen abgeleitete Substanzen sowie deren Anwendung in diagnostischen und therapeutischen Verfahren.

In einer bevorzugten Ausführungsform der diagnostischen und therapeutischen Verfahren erfolgt ein Nachweis bzw. eine Beeinflussung von neuronalen Erkrankungen, insbesondere neuronalen degenerativen Erkrankungen wie Morbus Alzheimer. So ist beispielsweise eine Verwendung bei der Verlaufskontrolle von neurodegenarativen Erkrankungen denkbar. Überraschenderweise wurde nämlich festgestellt, dass die Nervenzellen von Patienten mit Morbus Alzheimer eine insgesamte schwächer positive Reaktion mit Anti-Neuregulin-β-Antikörpern als Normalpersonen aufweisen.

Schließlich betrifft die Erfindung neue Neuregulin-β Isoformen, z.B. PTM-Modifikationen, mit einem sauren isoelektrischen Punkt, vorzugsweise im pH-Bereich von 4,3 bis 5,0. Die erfindungsgemäße Neuregulin-Isoform hat eine apparente Molekularmasse (SDS-PAGE) von etwa 32 kD und tritt nach Stimulation im erfindungsgemäßen Effekt/Effektraum-Analyseverfahren in erheblich höheren Mengen als ohne Stimulation oder bei Kontrollen auf. Das Protein enthält - wie durch massenspektroskopische Analyse nachgewiesen - Peptidteilfragmente entsprechend den Peptiden 81 bis 87 und 88 bis 94 von Neuregulin-β. Neben dem Protein mit vollständiger Sequenz erfasst die Erfindung auch physiologisch aktive Derivate und Fragmente davon. Derartige Derivate und Fragmente können durch rekombinante Expression entsprechender mutagenisierter Nukleinsäuresequenzen bzw. durch proteolytische Spaltung erhalten werden.

Die erfindungsgemäßen Neuregulin-β lsoformen können aus neuronalen Säugerzellen, z.B. Rattenzellen oder menschlichen Zellen, durch bekannte Methoden, z.B. Immunadsorption unter Verwendung geeigneter Antikörper gegebenenfalls in Kombination mit einer isoelektrischen Fraktionierung gewonnen werden.

Die nachfolgenden Beispiele und Figuren dienen zur Erläuterung der Erfindung.

Es zeigen:
- **Figur 1**: eine Aufsicht einer erfindungsgemäßen Fließzell-Vorrichtung,
- **Figur 2**: ein Querschnitt einer in Figur 1 gezeigten Fließzell-Vorrichtung,
- **Figur 3**: eine Aufsicht einer weiteren Fließzell-Vorrichtung.
- **Figur 4**: ein 2-D-PAGE von glutamat-induzierten Änderungen hippocampaler Proteine,
- **Figur 5**: eine Identifikation von Neuregulin-β durch MS/MS-Analyse mittels ESI-MS.

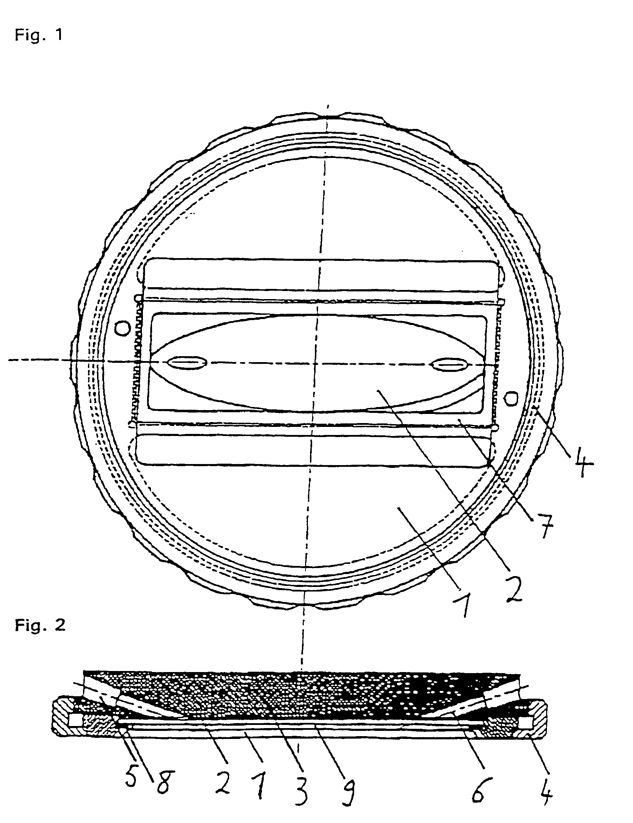

**Figur 1** ist eine Aufsicht einer erfindungsgemäßen Fließzell-Vorrichtung. Eine Grundplatte (1) weist einen Strömungskanal (2) auf. Auf der Grundplatte (1) liegt zentrosymmetrisch ein Verschlußring (4). Weiterhin ist ein auf der Grundplatte (1) liegender Objektträger-Aufnahmerahmen (7) zu erkennen.

**Figur 2** ist ein Querschnitt einer in Figur 1 gezeigten Fließzell-Vorrichtung. Dort sind neben den oben erwähnten Merkmalen weiterhin die folgenden zu erkennen: Der Verschlußring (4) klemmt die Grundplatte (1) mit einer Oberplatte (3) mit Hilfe einer Silikondichtung (8) abdichtend zusammen. (Nicht näher eingezeichnete) in beide Platten (1, 3) eingelassene Stifte sorgen für eine gewisse Beabstandung und somit Festlegung einer Kammerhöhe. Der nunmehr mittels der Oberplatte (3) von oben geschlossene Strömungskanal (2) bildet eine Kammer, die eigentliche Kammer, in der die Wechselwirkungen zwischen Zellkulturen und über Zu- und Ableitungen (5, 6) ein- und abfließenden Strömungsmedien stattfinden.

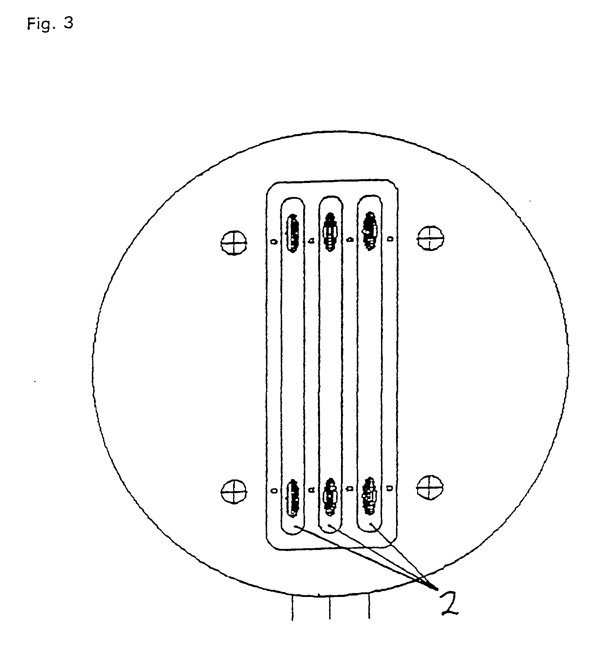

**Figur 3** ist eine Aufsicht einer weiteren Fließzell-Vorrichtung. Dort sind schematisch insgesamt drei Strömungskanäle (2) zu erkennen.

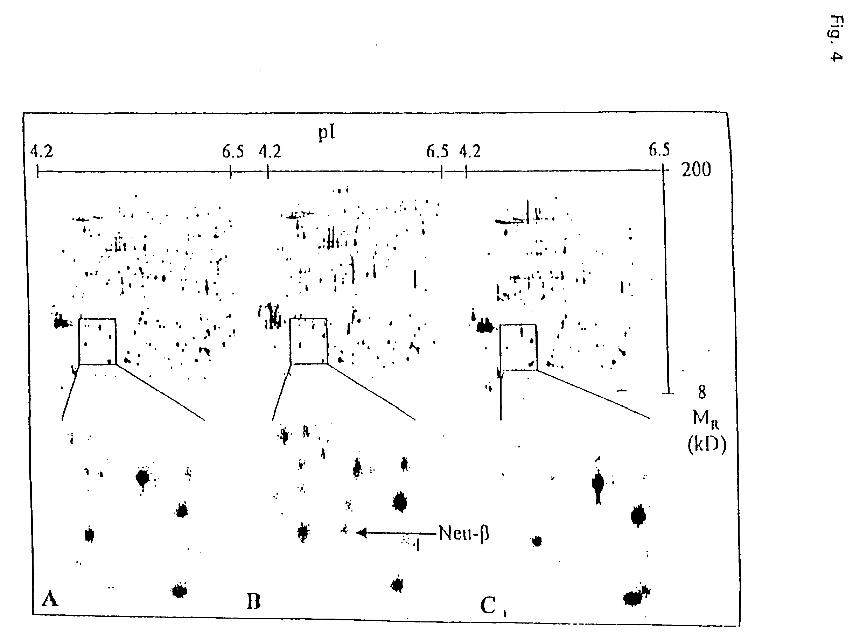

In **Figur 4** ist ein 2-D-PAGE von glutamat-induzierten Änderungen hippocampaler Proteine zu erkennen. Hippocampale Zellen wurden mit Stimulationspuffer stimuliert (in mM): NaCl (125), KCL (5), CaCl₂ (2-6), MgCl₂ (0,8), Glucose (5-10), HEPES (20), L-Glutamat (0,01-0,1), Glycin (0,01), Bicucullin (0,01), pH 7,2; (A = Kontrolle, B = Glutamat, C = Glutamat + MK-801; MK-801 ist ein spezifischer Glutamatrezeptor-Antagonist). Funktionelle Meß- bzw. Kontrollgröße war dabei ein mit Fura-2 gemessenes Fluoreszenz-Signal, welches proportional zur Calciumkonzentration ist (alle Stoffe sind in Chemikalienkatalogen von Torris oder Sigma nachlesbar).

Die auffälligste und konsistenteste Änderung ist mit einem Pfeil markiert: Neu-β (32 kDa, pl = 4,5 bis 4,7 (isoelektrischer Punkt)) ist in erheblich größerer Menge in der stimulierten Kondition zu beobachten und in den Kontrollen nur schwach oder gar nicht zu finden.

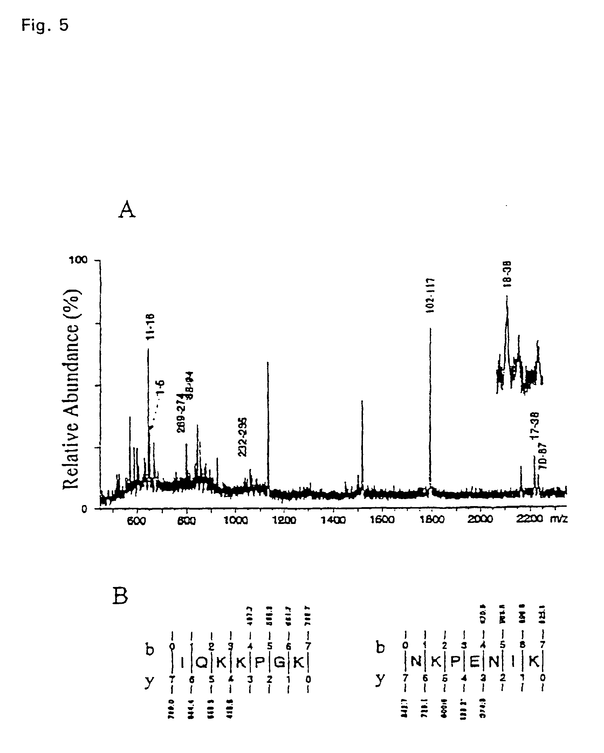

In **Figur 5** ist eine Identifikation von Neuregulin-β durch MS/MS-Analyse mittels ESI-MS zu erkennen.

Ausgewählte Peptide der MALDI-MS wurden zur Aminosäure-Sequenzierung mit ESI-MS fragmentiert. (MALDI, ESI-Sequenzierung wie in Soskic V., Görlach M, Poznanovic S., Boehmer FD, Godovac-Zimmermann J., "Functional proteomics analysis of signal transduction pathways of the platelet-derived growth factor beta receptor" (1999), Biochemistry 38, 1757-1764; oder "Proteome Research, Two-Dimensional Gel Electrophoresis and Identification Methods" von Thierry Rabilloud, 2000, Springer-Verlag, Berlin). Die erhaltenen Sequenzen identifizieren eindeutig ein Neuregulin-β (Details, siehe Tabelle 2).

### Beispiel 1 Identifizierung von Neuregulin-β als Indikator bzw. Target für neuronale Prozesse

### 1.1 Neuronale Zellkultur (aus Ratten-Hippocampus), LTP/LTD:

Geeignete Zellkultursysteme, im Falle des Neuregulin-β, hippocampale Primärkulturen aus prä- oder neonatalen Ratten (beschrieben in z.B. "Rat hippocampal neurons in low-density culture", K. Goslin, H. Asmussen, G. Banker; in "Culturing Nerve Cells, 2^{nd} edition 1998, S. 339 ff; K. Goslin and G. Banker, eds. MIT Press, Cambridge, Massachusetts, USA) werden auf den oben beschriebenen Objektträgern kultiviert, in die Flußzelle eingebracht und zunächst mikroskopisch charakterisiert (morphologische Aufnahmen etc. im Phasenkontrast).

Anschließend erfolgt die Inkubation der Zellen mit jeweils geeigneten Fluoreszenzfarbstoffen (für die gedächtnisrelevanten Versuche an Neuronen, die zur Entdeckung der besonderen Funktion des Neuregulin-β geführt haben: Fura-2, ein calciumsensitiver Fluoreszenzfarbstoff).

### 1.2 Effektraum/Effektorraum-Analyse

Danach werden die Zellen über das computer-gesteuerte Applikationssystem (für die gedächtnisrelevanten Versuche an Neuronen, die zur Entdeckung des Neuregulin-β geführt haben: 100 µM Glutamat für 30 Sekunden) stimuliert.

Es erfolgt eine zeitlich aufgelöste Erfassung der Fluoreszenzsignale in einer großen Zahl von Zellen in Abhängigkeit von den Stimulationsbedingungen (Temperatur, Wirkstoff). Im Zusammenhang mit Neuregulin-β löst Glutamat einen Einstrom von Calcium-Ionen aus, der sich über die Wechselwirkung mit Fura-2 sehr empfindlich quantifizieren läßt.

Es findet quasi parallel dazu eine synchronisierte Fraktionierung und Analyse des Perfusates statt; im Falle von hippocampalen Neuronen von Neurotransmittern mittels HPLC mit Fluoreszenz-Detektion, im Falle von anderen niedermolekularen extrazellulären Komponenten mittels EI-MS und im Falle von stabilen lsotopen-Tracern und Metallionen mittels ICP-MS.

Darüber hinaus ist eine lmmunfärbung der Zellen mit spezifischen Antikörpern gegen Oberflächenmarker zur weitergehenden Charakterisierung möglich.

Weiterhin findet eine Charakterisierung und Identifizierung von Proteinen statt, die sich unter den gewählten experimentellen Bedingungen verändern oder sonst auffällig verhalten, mit den Techniken der Proteom-Forschung, nämlich mittels zwei-dimensionaler Gelelektrophorese, MALDI-TOF-MS und ESI-MS.

Schließlich findet eine Integration, d.h. eine Zusammenfassung, der gewonnenen multidimensionalen Funktionsdaten für die Effektraum/Effektorraum-Analysevon Wirkstoffen statt. Die Effektraum-/Effektorraum-Analyse ist eine Korrelationsanalyse bezüglich der einzelnen Vorgänge und den damit einhergehenden Konzentrationsänderungen bestimmter Stoffe.

Die folgenden Parameter wurden in dem beschriebenen Aufbau simultan bzw. eindeutig korrelierend bestimmt. Die Steigerung des Calcium-Einstroms in Neuronen hippocampalen Ursprungs (Beweis der neuronalen Identität durch die tau-Protein-Färbung derselben Zellen nach dem funktionellen Teil des Experiments) bei gleichbleibender Temperatur identifizieren den physiologischen Kontext (LTP-(Gedächtnis)-relevant). Damit einhergehend sind die gleichzeitigen Änderungen der Neurotransmitter-Freisetzung (GABA), des durch den stabilen Isotopen-Tracer ²⁶Mg nachgewiesenen Mg-Einstroms in die Zellen. Insbesondere das Auftreten von Neuregulin-β in der stimulierten Kondition ist ein neues und unerwartetes Ergebnis. Dieser Satz von Informationen ist dadurch erstmalig in einem gedächtnis-relevanten Zusammenhang erkannt worden. Neuregulin-β ist somit auch als potentielles Target für Wirkstoffe identifiziert, die in bezug auf Gedächtniskrankheiten getestet werden sollen. Mit dem vorgestellten Aufbau wird eine wesentlich höhere Informationsdichte erreicht als in herkömmlichen Screening-Verfahren, wo meist nur einzelne Parameter in Verbindung mit Wirkstoffen untersucht werden.

Der stabile Isotopen-Tracer ist im Anwendungsbeispiel ²⁶Mg. Zur Immunfärbung wurde ein anti-tau-Antikörper (beispielsweise durch SIGMA zu erhalten) verwendet.

Die Identifizierung von Neuregulin-β erfolgte durch zweidimensionale PAGE unter Verwendung isoelektrischer Fokussierung mit immobilisierten nichtlinearen pH-Gradienten von 3 bis 10 und linearen Gradienten von 4 bis 7 als erste Dimension und SDS-PAGE (12 % Acrylamid) als zweite Dimension. Eine Silberanfärbung der Gele zeigte ein reproduzierbares Muster von mehreren tausend Proteinen, wobei die meisten unter allen Bedingungen unverändert blieben. Der auffälligste Unterschied war ein Protein mit einer apparenten Molekularmasse von etwa 32 kD und einem pl-Wert von 4,5 bis 4,7, das nach Stimulation in erheblich höheren Mengen als ohne Stimulation oder bei den Kontrollen (Zusatz von 10 µM MK-801, einem spezifischen Inhibitor von NMDA-Rezeptoren) (Wong et al, Proc. Natl. Acad. USA 83 (1986), zu, 7104-7106).

Das Protein wurde aus den Gelen herausgeschnitten, einer Trypsinspaltung innerhalb des Gels unterzogen und anschließend massenspektroskopisch durch MALDI-TOF-Massenfingerprint und ESI-Peptidsequenzierung (Corthals et al., Identification of Proteins by Mass-Spectrometry, in: Proteome Research, Two-Dimensional Gel-Electrophoresis and Identification Methods, Thierry Rabilloud, Hrsg., Springer, Berlin (2000), 197-232).

Die Identität des Proteins wurde durch Untersuchung ausgewählter Peptide mit MS/MS-Analyse unter Verwendung von lonenfallen, Elektrospray, Massenspektroskopie des nicht aufgetrennten, zuvor durch MALDI-TOFanalysierten Peptidgemisches bestätigt. Die MS/MS-Analyse von m/z 798.4 war ausreichend, um u.a. das Peptid 88-94 von Neuregulin-β zu identifizieren. Die MS/MS-Analyse von m/z = 842,7 bestätigte die Sequenz des Peptids um Position 81 - 87. Diese Peptidsequenzen sind den den Neuregulinen α und β konserviert, im zentralen Nervensystem sind jedoch Neureguline-β prädominant (Meyer et al., Development 124 (1997), 3575-3586). Die experimentellen Bedingungen und die Resultate sind in den Tabellen 1 und 2 dargestellt.

Der aus der Aminosäuresequenz von Neuregulinen berechnete pl beträgt etwa 9,0, während das im vorliegenden Experiment identifizierte Neuregulin-β Isoform einen pl im Bereich von 4,3 bis 5,0 aufweist, was auf umfangreiche posttranslationale Modifikationen hinweist.

Die vorliegenden Ergebnisse zeigen, dass Neuregulin-β durch physiologische Aktivität reguliert wird und eine wichtige Rolle bei der neuralen Plastizität spielt. Weiterhin ist anzunehmen, dass die NDF-Rezeptor-Tyrosinkinasen ErbB-3 und ErbB4 über Neuregulin-β an der Induzierung von LTP beteiligt sind.

### Beispiel 2 Identifizierung von Neuregulin-β als Target bei Morbus Alzheimer

Schnittpräparate (3 von Patienten mit normalem Hirnbefund und 3 von Patienten mit Morbus Alzheimer) wurden immunhistochemisch mit Hilfe der ABC-Methode untersucht, wobei das Aminobenzidin als Substrat diente. Der Anti-Neuregulin-Primärantikörper (Santa Cruz Biotech) wurde in zwei Verdünnungen vorher ausgetestet (1:10 und 1:100). Als Negativkontrolle wurde ein unspezifisches Ziegenserum anstelle des Primärantikörpers eingesetzt.

Bei den Präparaten aus Normalgehirn wurden hippocampale Strukturen mit Nervenzellen gefunden, die eine schwache Anfärbung mit dem Antikörper im Cytoplasma aufweisen. Zellen des Plexus Choriodeus zeigten eine deutlich positive Reaktion im Cytoplasma.

Im Hirngewebe von Patienten mit Morbus Alzheimer wurden hippokampale Strukturen mit Nervenzellen gefunden, die eine deutlich schwächere Reaktion mit dem Antikörper im Cytoplasma aufweisen. Zellen des Plexus Choriodeus weisen eine positivere Reaktion im Cytoplasma auf.

Insgesamt scheinen die Nervenzellen von Patienten mit Morbus Alzheimer eine schwächer positive Reaktion mit Anti-Neuregulin-β-Antikörpern als Normalpersonen aufzuweisen.

**Tabelle 1:**

| **Parameter** | **Konzentration/Expression/Meßgröße (vorstimuliert)** | **Konzentration/Expression/Meßgröße der Kontrolle** | **Methode** |
|---|---|---|---|
| GABA | 74 pMol | 87 pMol | Fluoreszenz-HPLC |
| Neuregulin-β | stark exprimiert | nicht exprimiert | 2-D PAGE, ESI-MS, MALDI-TOF-MS |
| ²⁶Mg (intrazellulär) | 45 nM/mg Protein | 25 nM/mg Protein | ICP-MS |
| Calcium (intrazellulär) | 850 nM | 350 nM | Fura-2; Fluoreszenz-Imaging |
| tau-Protein (neuronaler Marker) | positiv | positiv | Immunfärbung mit anti-tau-Antikörper |
| Temperatur | +22°C | +22°C | intrinsisch |

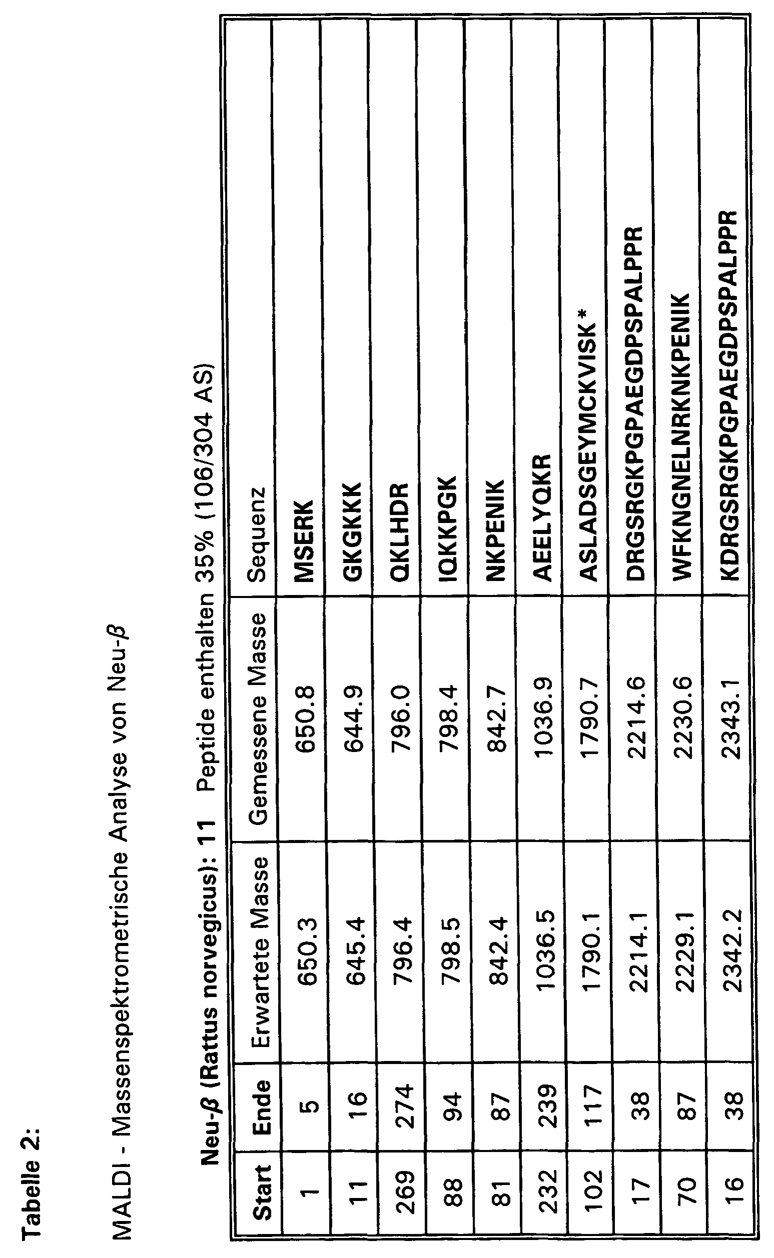

## Patentansprüche

1. Neuregulin-β Isoform mit einem sauren isoelektrischen Punkt (pl) und einer apparenten Molekularmasse von etwa 32 kDa (gemäß 2D-PAGE unter Verwendung von isoelektrischer Fokussierung mit immobilisierten nichtlinearen pH-Gradienten von 4-7 als erste Dimension und SDS-PAGE, 12 % Acrylamid, als zweite Dimension), die mit Gedächtnis-Krankheiten assoziiert ist.

2. Neuregulin-β Isoform nach Anspruch 1 mit einem pl im Bereich von pH 4,3 bis 5,0.

3. Neuregulin-β Isoform nach Anspruch 1 oder 2 mit einem pl im Bereich von pH 4,5 bis 4,7.

4. Verwendung von Neuregulin-β nach Anspruch 1, 2 oder 3 als Indikator und/oder Target in einem Screening-Verfahren zur Identifizierung von Wirkstoffen für den Nachweis und/oder die Beeinflussung neuronaler Prozesse.

5. Verwendung von Neuregulin-β nach Anspruch 1, 2, oder 3 als Indikator und/oder Target in einem Screening-Verfahren zur Identifizierung von Wirkstoffen zur Beeinflussung von Glutamatrezeptor-vermittelten Calcium-Konzentrationsänderungen.

6. Verwendung von Neuregulin-β nach Anspruch 1, 2, oder 3 als Indikator und/oder Target in einem Screening-Verfahren zur Identifizierung von Wirkstoffen, wobei das Screening-Verfahren ein Verfahren zur Erfassung von multidimensionalen Funktionsdaten für die Effektraum-/Effektoraum-Analyse von Wirkstoffen ist,
- bei dem in eine Fließzell-Vorrichtung eingebrachte und auf Substraten aufgebrachte Zellkultursysteme mit jeweils geeigneten Fluoreszenzfarbstoffen und/oder mit Isotopengemischen verschiedener Elemente inkubiert,
- die Zellkultursysteme stimuliert und
- zeitlich synchron die in Abhängigkeit von den Stimulationsbedingungen resultierenden Fluoreszenzsignale direkt am Zellkultursystem zeitlich aufgelöst erfasst und/oder die Isotopengemische im Perfusat zeitlich aufgelöst erfasst und/oder die Isotopengemische direkt an den Zellkultursystemen zeitlich integral erfasst werden,
- wobei eine synchronisierte Fraktionierung und weitergehende Analyse des Perfusates,
- eine Charakterisierung und Identifizierung von sich unter den gewählten Bedingungen verändernden und/oder spezifisch verhaltenden Proteinen der Zellkultursysteme und
- schließlich eine Integration der gewonnenen multidimensionalen Funktionsdaten für die Effektraum/Effektorraum-Analyse von Wirkstoffen vorgenommen wird.

7. Verwendung nach einem der Ansprüche 4 bis 6,
**dadurch gekennzeichnet,**
**dass** das Neuregulin-β als Indikator und/oder Target zur Beeinflussung von neuronalen Prozessen eingesetzt wird.

8. Verwendung nach Anspruch 7,
**dadurch gekennzeichnet,**
**dass** das Neuregulin β als Indikator und/oder Target zur Beeinflussung von Langzeitverstärkung (LTP), Langzeitdepression (LTD), epileptophormen und/oderepileptogenen Ereignissen und/oder erregungs-toxischen Phänomenen eingesetzt wird.

9. Verwendung nach einem der Ansprüche 4 bis 6,
**dadurch gekennzeichnet,**
**dass** das Neuregulin-β als Indikator und/oder Target zur Beeinflussung von neuronalen Erkrankungen, und/oder neuronalen degenerativen Erkrankungen eingesetzt wird.

10. Verwendung nach Anspruch 9,
**dadurch gekennzeichnet,**
**dass** das Neuregulin β als Indikator und/oder Target zur Beeinflussung von Morbus Alzheimer eingesetzt wird.

11. Verwendung nach einem der Ansprüche 4 bis 10,
**dadurch gekennzeichnet,**
**dass** das Neuregulin-β als Indikator und/oder Target zur Beeinflussung von durch Glutamatrezeptoren vermittelten Calcium-Konzentrationsänderungen eingesetzt wird.

12. Verwendung von Neuregulin-β nach Anspruch 1, 2, oder 3 zum Screening für Wirkstoffe und/oder zur Ermittlung von Indikatoren und/oder Targets für den Nachweis und/oder die Beeinflussung neuronaler Prozesse.

13. Verwendung von Neuregulin-β nach Anspruch 1, 2, oder 3 als Indikator in einem Verfahren zum Nachweis neuronaler Prozesse.

14. Mittel, umfassend Neuregulin-β nach Anspruch 1, 2 oder 3 als Target in einem Verfahren zur Beeinflussung neuronaler Prozesse.

15. Mittel, umfassend Neuregulin-β nach Anspruch 14 zum Nachweis bzw. zur Beeinflussung von neuronalen Erkrankungen bzw. zur Verwendung bei der Verlaufskontrolle von neuronalen degenerativen Erkrankungen.

16. Mittel, umfassend Neuregulin-β nach Anspruch 14, wobei es sich bei der neuronalen degenerativen Erkrankung um Morbus Alzheimer handelt.

## Claims

1. Neuregulin-β isoform having an acidic isoelectric point (pI) and an apparent molecular mass of about 32 kDa (according to 2D-PAGE using isoelectric focussing with immobilized non-linear pH gradients of 4-7 as the first dimension and SDS-PAGE, 12 % acrylamide, as the second dimension) which is associated with memory diseases.

2. Neuregulin-β isoform as claimed in claim 1 having a pI in the range from pH 4.3 to 5.0.

3. Neuregulin-β isoform as claimed in claim 1 or 2 having a pI in the range from pH 4.5 to 4.7.

4. Use of neuregulin-β as claimed in claim 1, 2 or 3 as an indicator and/or target in a screening method to identify active substances for detecting and/or for influencing neuronal processes.

5. Use of neuregulin-β as claimed in claim 1, 2 or 3 as an indicator and/or target in a screening method to identify active substances for influencing glutamate receptor-mediated changes in calcium concentration.

6. Use of neuregulin-β as claimed in claim 1, 2 or 3 as an indicator and/or target in a screening method to identify active substances, the screening method being a method for collecting multi-dimensional functional data for the effect space/effector space analysis of active substances,
- in which cell culture systems that have been introduced into a flow cell device and applied to substrates are incubated with fluorescent dyes that are in each case suitable and/or with isotopic mixtures of different elements,
- the cell culture systems are stimulated, and
- synchronously, the fluorescence signals resulting in dependence on the stimulation conditions are detected directly in the cell culture system in a time-resolved manner, and/or the isotopic mixtures are detected in the perfusate in a time-resolved manner, and/or the isotopic mixtures are detected directly in the cell culture systems in a time-integrated manner,
- during which a synchronized fractionation and further analysis is carried out on the perfusate,
- proteins in the cell culture systems which change and/or behave specifically under the selected conditions are **characterized** and identified, and
- finally the multi-dimensional functional data that have been obtained are integrated for the effect space/effector space analysis of active substances.

7. Use as claimed in one of the claims 4 to 6,
**characterized in that**
the neuregulin-β is used as an indicator and/or a target for influencing neuronal processes.

8. Use as claimed in claim 7,
**characterized in that**
neuregulin-β is used as an indicator and/or target for influencing long-term potentiation (LTP), long-term depression (LTD), epileptiform and/or epileptogenic events and/or excitation-toxic phenomena.

9. Use as claimed in one of claims 4 to 6,
**characterized in that**
neuregulin-β is used as an indicator and/or target for influencing neuronal diseases and/or neuronal degenerative diseases.

10. Use as claimed in claim 9,
**characterized in that**
neuregulin-β is used as an indicator and/or target for influencing Alzheimer's disease.

11. Use as claimed in one of claims 4 to 10,
**characterized in that**
neuregulin-β is used as an indicator and/or target for influencing changes in calcium concentration that are mediated by glutamate receptors.

12. Use of neuregulin-β as claimed in claim 1, 2 or 3 to screen for active substances and/or to determine indicators and/or targets for detecting and/or influencing neuronal processes.

13. Use of neuregulin-β as claimed in claim 1, 2 or 3 as an indicator in a method for detecting neuronal processes.

14. Agent comprising neuregulin-β as claimed in claims 1, 2 or 3 as a target in a method for influencing neuronal processes.

15. Agent comprising neuregulin-β as claimed in claim 14 for detecting or influencing neuronal diseases or for use in monitoring the course of neuronal degenerative diseases.

16. Agent comprising neuregulin-β as claimed in claim 14 wherein the neuronal degenerative disease is Alzheimer's disease.

## Revendications

1. Isoforme de neuréguline-β ayant un point isoélectrique (pI) acide et une masse molaire apparente d'environ 32 kDa (selon une PAGE en 2D utilisant une focalisation isoélectrique avec des gradients de pH non linéaires immobilisés de 4-7 comme première dimension et une SDS-PAGE, acrylamide à 12 %, comme deuxième dimension), qui est associée à des maladies de la mémoire.

2. Isoforme de neuréguline-β selon la revendication 1, ayant un pI dans le domaine de pH 4,3 à 5,0.

3. Isoforme de neuréguline-β selon la revendication 1 ou 2, ayant un pI dans le domaine de pH 4,5 à 4,7.

4. Utilisation de neuréguline-β selon la revendication 1, 2 ou 3 comme indicateur et/ou cible dans un procédé de criblage pour l'identification de substances actives destinées à détecter et/ou à influencer des processus neuronaux.

5. Utilisation de neuréguline-β selon la revendication 1, 2 ou 3 comme indicateur et/ou cible dans un procédé de criblage pour l'identification de substances actives destinées à influencer des variations de concentration de calcium médiées par le récepteur de glutamate.

6. Utilisation de neuréguline-β selon la revendication 1, 2 ou 3 comme indicateur et/ou cible dans un procédé de criblage pour l'identification de substances actives, le procédé de criblage étant un procédé de détermination de données fonctionnelles à plusieurs dimensions pour l'analyse de l'espace d'effets/espace d'effecteurs de substances actives, dans lequel
- on met à incuber des systèmes de cultures de cellules introduits dans un dispositif de cellule à écoulement et appliqués sur des substrats avec à chaque fois des colorants de fluorescence appropriés et/ou avec des mélanges d'isotopes de différents éléments,
- on stimule les systèmes de culture de cellules, et
- de façon temporellement synchrone, on détermine de manière séparée dans le temps les signaux de fluorescence résultants en fonction des conditions de stimulation directement sur les systèmes de culture de cellules et/ou on détermine de manière séparée dans le temps les mélanges d'isotopes dans le perfusat, et/ou on détermine de manière intégrale dans le temps les mélanges d'isotopes directement sur les systèmes de culture de cellules, en effectuant
- un fractionnement synchronisé et une analyse plus poussée du perfusat,
- une caractérisation et une identification des protéines des systèmes de culture de cellules qui se transforment et/ou qui se comportent de façon spécifique dans les conditions choisies, et
- enfin une intégration des données fonctionnelles à plusieurs dimensions obtenues pour l'analyse de l'espace d'effets/espace d'effecteurs de substances actives.

7. Utilisation selon l'une des revendications 4 à 6, **caractérisée en ce que** l'on utilise la neuréguline-β comme indicateur et/ou cible pour agir sur des processus neuronaux.

8. Utilisation selon la revendication 7, **caractérisée en ce que** l'on utilise la neuréguline-β comme indicateur et/ou cible pour agir sur la potentialisation à long terme (LTP), la dépression à long terme (LTD), les évènements épileptoformes et/ou épileptogènes et/ou les phénomènes toxiques d'excitation.

9. Utilisation selon l'une des revendications 4 à 6, **caractérisée en ce que** l'on utilise la neuréguline-β comme indicateur et/ou cible pour agir sur des maladies neuronales et/ou des maladies neuronales dégénératives.

10. Utilisation selon la revendication 9, **caractérisée en ce que** l'on utilise la neuréguline-β comme indicateur et/ou cible pour agir sur la maladie d'Alzheimer.

11. Utilisation selon l'une des revendications 4 à 10, **caractérisée en ce que** l'on utilise la neuréguline-β comme indicateur et/ou cible pour agir sur des variations de concentration de calcium médiées par les récepteurs de glutamate.

12. Utilisation de neuréguline-β selon la revendication 1, 2 ou 3 pour le criblage de substances actives et/ou pour la recherche d'indicateurs et/ou de cibles destinés à détecter et/ou influencer des processus neuronaux.

13. Utilisation de neuréguline-β selon la revendication 1, 2 ou 3 comme indicateur dans un procédé de détection de processus neuronaux.

14. Agent contenant de la neuréguline-β selon la revendication 1, 2 ou 3 comme cible dans un procédé pour agir sur des processus neuronaux.

15. Agent comprenant de la neuréguline-β selon la revendication 14 pour détecter ou influencer des maladies neuronales, ou à utiliser dans le contrôle de l'évolution de maladies neuronales dégénératives.

16. Agent comprenant de la neuréguline-β selon la revendication 14, la maladie neuronale dégénérative étant la maladie d'Alzheimer.
